# EUROPÄISCHE PATENTSCHRIFT

(11) **EP 0 855 032 B1**
(45) Veröffentlichungstag und Bekanntmachung des Hinweises auf die Patenterteilung: **21.08.2002**
(21) Anmeldenummer: 96933422.6
(22) Anmeldetag: 30.09.1996
(51) Int. Cl.: G01N 33/571

(54) **VERFAHREN UND MITTEL ZUM NACHWEIS VON ANTIKÖRPERN GEGEN TREPONEMA PALLIDUM**
PROCESS AND AGENT FOR DETECTING ANTIBODIES AGAINST TREPONEMA PALLIDUM
PROCEDE ET DISPOSITIF D'IDENTIFICATION D'ANTICORPS CONTRE TREPONEMA PALLIDUM

(30) Priorität: 29.09.1995 DE 19536166
(43) Veröffentlichungstag der Anmeldung: 29.07.1998
(73) Patentinhaber: Krell, Siegfried, 39108 Magdeburg (DE)
(72) Erfinder: KRELL, Siegfried, D-39108 Magdeburg (DE); GERBER, Annegret, D-39108 Magdeburg (DE)
(74) Vertreter: Einsel, Martin, Dipl.-Phys.
(86) Internationale Anmeldenummer: EP9604249
(87) Internationale Veröffentlichungsnummer: WO97013151

(56) Entgegenhaltungen:
- EP-A- 0 391 330
- EP-A- 0 670 494
- EP-A- 0 686 696
- WO-A-88/02403
- US-A- 5 350 842
- INFECTION AND IMMUNITY, Bd. 61, Nr. 4, 1993, Seiten 1202-1210, XP002024431 DARRIN R. AKINS: "LIPID MODIFICATION OF THE 17-KILODALTON MEMBRANE IMMUNOGEN OF TREPONEMA PALLIDUM DETERMINES MACROPHAGE ACTIVATION AS WELL AS AMPHIPHILICITY."

## Beschreibung

Die Erfindung betrifft ein Verfahren und ein Mittel zum Nachweis von Antikörpern gegen *Treponema pallidum*.

*Treponema pallidum* ist der Erreger der Syphilis. Eine Diagnosestellung für Infektionen mit diesem Krankheitserreger ist nicht nur in Verdachtsfällen wichtig; von großer Bedeutung ist es, eine Syphilis zum einen bei Schwangeren und zum anderen bei Blutspendern auszuschließen. In der Schwangerenvorsorge dieht dies der Prävention einer konnatalen Syphilis, also der Übertragung der Syphilis auf das Neugeborene und dessen möglicher Schädigung. Bei Blutspendem ist eine negative Syphilisserologie ein Spenderauswahlkriterium.

Die Syphilisdiagnostik erfolgt in erster Linie serologisch, d.h. durch den Nachweis von Antikörpem gegen *Treponema pallidum* und/oder Cardiolipin. Bereits 14 Tage nach Infektion sind spezifische IgM- und spätestens 4 Wochen nach Infektion IgG-Antikörper nachweisbar. Nur in der davorliegenden Zeit ist direkter Erregemachweis aus dem infizierten Gewebe des Primäraffektes ein weiteres entscheidendes diagnostisches Kriterium.

Eine Diagnosestellung durch kulturellen Nachweis, wie sonst für viele andere bakterielle Krankheitserreger üblich, ist nicht möglich, da eine *in vitro*-Kultur von *Treponema pallidum* bisher nicht gelungen ist, abgesehen von einer Passagierung auf Zellkulturen (US-PS 5,264,360).

Die Nachweismethoden für die serologische Diagnostik lassen sich nach der Natur der nachgewiesenen Antikörper in drei Gruppen einteilen:
1. Nicht-Treponemen-spezifische Tests. Auf dem Nachweis von Antikörpern gegen Cardiolipin basieren Tests wie der Cardiolipin- Mikroflockungstest (CMT), der im englischsprachigen Raum als Veneral Disease Laboratory Test (VDRL-Test) bezeichnet wird, der "Rapid Plasma Reagin Test" (RPR-Test) und die Cardiolipin-Komplementbindungsreaktion (Cardiolipin-KBR). Diese Tests werden 3-5 Wochen nach Infektion bzw. etwa 7-10 Tage nach Erscheinen des Primäraffektes positiv. Die Sensitivität beträgt 60 bis 87 % im Primärstadium und kann 100 % bei Sekundärsyphilis erreichen. Die Sensitivität fällt jedoch im weiteren Krankheitsverlauf, so daß bis zu 30 % der Spätstadien nicht mehr reaktiv sind. Bei quantitativer Durchführung des VDRL-Tests ist eine Titerabhängigkeit von der Krankheitsaktivität zu verzeichnen. Nachteil dieser Tests ist der hohe Anteil von 0,3-0,9 % falsch positiven Testergebnissen bei Untersuchung von Blutspenderseren sowie das Vorkommen falsch negativer Ergebnisse bei hochtitrigen Seren infolge Prozonenphänomens, das bei Sekundärsyphilis im VDRL-Test in 1 bis 2 % der Fälle beobachtet werden kann.
2. Treponemen-spezifische Tests. Im Verlauf der Syphilis werden gegen Endoflagellen von *Treponema pallidum* Antikörper gebildet. Auf Grund von Antigenverwandtschaften reagieren diese Antikörper auch mit Endoflagellen anderer Treponemenspecies. Für die Syphilisdiagnostik wurden deshalb auch Endoflagellen von *Treponema phagedenis* (Biotyp Reiter) als Antigen eingesetzt (Flagellum-ELISA. R. V. W. Van Eijk et al., Genitourin. Med. 62, 367-372 (1988)). Beim Flagellum-ELISA stellt der Grenzwert für ein positives Testergebnis einen Kompromiß bezüglich Sensitivität und Spezifität dar. Damit sind 0,8 % falsch positive und 2,7 % falsch negative Testergebnisse zu verzeichnen.
3. *Treponema-pallidum-*spezifische Tests. Diese Tests weisen Antikörper nach, die mit *Treponema pallidum* oder Antigenpräparationen aus dem Erreger reagieren. Zu diesen Testsystemen gehören der *Treponema-pallidum-*Hämagglutinationstest (TPHA), der Fluoreszenz-*Treponema-pallidum*-Antikörper-Absorptionstest (FTA-ABS) und der Nelson-Test *(Treponema pallidum*-Immobilisationstest, TPI) sowie ELISA-Systeme auf der Basis von Sonikatantigen. In der Diagnostik werden vorrangig TPHA und FTA-ABS eingesetzt.

Beim Nelson-Test wird mikroskopisch beurteilt, wie komplementaktivierende Antikörper im Patientenserum die Beweglichkeit von *Treponema pallidum* hemmen. Der Nelson-Test wird wegen des hohen Aufwandes fast nur noch zu wissenschaftlichen Fragestellungen eingesetzt.

Beim TPHA werden Erythrozyten, an die *Treponema pallidum* -Sonikatantigen gebunden ist, durch Antikörper aus Syphilispatientenseren agglutiniert. Der TPHA weist mit 0,07 % falsch positiven und 0,008 % falsch negativen Ergebnissen eine hohe Spezifität und Sensitivität auf, wobei die meisten falsch negativen Ergebnisse die Frühphase der Syphilis betreffen. Der TPHA wird in der 4. Woche der Infektion positiv, zeigt bei Primärsyphilis eine Sensitivität von 64% bis 87 % mit zunächst geringer Titerhöhe (80 - 320), die beim Übergang zur Sekundärsyphilis auf über 5000 bei einer Sensitivität von 100 % ansteigen kann. Im weiteren Verlauf bleibt der TPHA positiv bei einer Tendenz zum Titerabfall im latenten Stadium.

Beim FTA-ABS wird die Bindung von spezifischen Antikörpern im Untersuchungsserum an objektträgerfixierte *Treponema pallidum* über einen fluoreszenzmarkierten Sekundärantikörper in der indirekten Immunfluoreszenzmikroskopie nachgewiesen. Die Sensitivität des Nachweises beträgt bei Primärsyphilis 86 % bis 100 %, erreicht bei Sekundärsyphilis 100 % und in den Spätstadien 96 % bis 100 %. Die Sensitivität für alle Stadien liegt verschiedenen Angaben zufolge zwischen 83 % und 95 %. Die Spezifität ist mit 83 % bis 89 % (ohne "borderline"-Befunde) nicht sehr hoch. Isoliert FTA-positive Seren bei negativer übriger Syphilisserologie sind z. B. durch Lyme-Borreliose bedingt. Der FTA-ABS wird als Bestätigungstest zur Absicherung positiver TPHA-Befunde durchgeführt.

Veldkamp und Visser (Brit. J. Vener. Dis. 51, 227-231 (1975)) beschrieben erstmalig einen ELISA (enzyme linked immunosorbent assay), bei dem Sonikatantigen von *Treponema pallidum*, das adsorptiv an Festphasen gebunden ist, mit spezifischen Antikörpem aus dem Untersuchungsmaterial reagiert. Der Nachweis der gebundenen Antikörper erfolgt durch Reaktion mit einem enzymmarkierten speziesspezifischen Sekundärantikörper gegen Immunglobuline der Klasse IgG bzw. IgM und nachfolgender enzymatischer Farbreaktion. Auf diesem Prinzip beruht auch der kommerzielle indirekte Enzymimmuntest Captia Syphilis G (Mercia Diagnostics, Guildford, England). Für diesen Test wurde eine Sensitivität von 98,4 % und eine Spezifität von 99,3 % ermittelt (Young et al., J. Clin. Pathol. 45, 37-41, (1992), Genitourin. Med. 65, 72-78 (1989)). Beim Captia Syphilis M wird spezifisches IgM nachgewiesen, das bereits in der Frühphase der Erkrankung gebildet wird (Sensitivität bei Primärsyphilis 82 %). Da IgM-Antikörper nicht placentagängig sind, ist dieser Test bedeutsam für die Diagnostik der konnatalen Syphilis (Sensitivität 100 %, Ijsselmuiden et al., J. Clin. Microbiol. 27, 152-157, (1989)). Das Testprinzip beruht auf einem festphasegebundenen Fangantikörper für IgM aus dem Untersuchungsmaterial, wobei spezifische IgM-Antikörper anschließend mit *Treponema pallidum-*Antigen reagieren. Der Nachweis erfolgt über einen enzymmarkierten monoktonalen Antikörper gegen Endoflagellen durch nachfolgende enzymatische Farbreaktion.

Die EP-A-0 670 494 beschreibt die Genamplifizierung, Klonierung, Expression und Reinigung der 15K und 17K Antigene aus Treponema Pallidum sowie deren Verwendung zur Bestimmung von Treponema Pallidum Antikörpern mittels eines Festphasenassays.

Bei dem Syphilis BioEnza Bead Assay (Organon Teknika Corp., USA) ist das *Treponema pallidum-*Antigen adsorptiv an ferromagnetische Partikel gebunden. Die Bindung von Antikörpem aus dem Untersuchungsmaterial wird dann über enzymmarkierten Sekundärantikörper und Farbreaktion nachgewiesen. Sensitivität und Spezifität werden für diesen Test mit 93 % und 98,6 % angegeben (Burdash et al., J. Clin. Microbiol. 25, 808-811, (1987)).

Sonikatantigen, wie vorstehend erwähnt, wird durch mechanische Aufarbeitung des Erregers mittels Ultraschall gewonnen.

Die oben schon erwähnte mangelnde *in vitro*-Kultivierbarkeit von *Treponema pallidum* führt natürlich dazu, daß diese Erreger nur sehr schwer zur Verfügung gestellt werden können.

Allgemein anerkannt ist derzeit eine zweistufige Diagnostik der Syphilis. In der ersten Stufe werden alle Seren mit einem Test untersucht (Screening). In der zweiten Stufe werden Seren mit positivem Befund mit einem anderen Testsystem untersucht (Bestätigungstest). Da die Prävalenz der Syphilis in vielen Ländern gering ist (< 1%), reicht eine 99%ige Spezifität des Screeningtests nicht zur Diagnosesicherung aus. Ein positiver Vorhersagewert (predictive value) von über 90 % wird erst mit einem Bestätigungstest, der auf andersartigem Nachweisprinzip beruht, erreicht.

Weit verbreitet ist die Anwendung von TPHA allein oder in Kombination mit VDRL-Test bzw. RPR-Test im Screening und die Prüfung der positiv gefundenen Seren im FTA-ABS als Bestätigungstest.

Als Therapiekontrolle ist die quantitative Durchführung von VDRL-Test bzw. RPR-Test anerkannt, wobei ein Titerabfall um mehr als vier Titerstufen als Therapieerfolg gewertet wird.

Bewertung der gegenwärtigen Syphilisdiagnostik : Nicht-Treponemen-spezifische Tests liefem ca. 5 % falsch positive Resultate. Sie sind daher für die serologische Diagnostik nur als erster Schritt geeignet und müssen bei positivem Ausfall im zweiten Schritt durch *Treponema-pallidum-*spezifische Tests ergänzt werden. Da Nicht-Treponemen-spezifische Tests bei quantitativer Auswertung eine Korrelation zur Krankheitsaktivität zeigen, können sie zur Erfolgskontrolle antibiotischer Therapie herangezogen werden.

Falsch positive Testergebnisse bei den treponemenspezifischen Tests sind wegen Antigenverwandtschaft zu Treponemen möglich, die beim Menschen als Kommensalen in der Mundschleimhaut vorkommen. Auf Grund der mangelhaften Spezifität und Sensitivität konnten sich diese Testsysteme zur Diagnostik der Syphilis nicht durchsetzen.

Die *Treponema pallidum*-spezifischen Tests basieren auf Antigenpräparationen von *Treponema pallidum,* wobei das verwendete Erregermaterial wegen der mangelnden *in vitro*-Kultivierbarkeit von *Treponema pallidum* aus infizierten Kaninchenhoden gewonnen wird. Das ist mit Fragen des Tierschutzes belastet und nur in geringer Menge möglich. Der FTA-ABS und der Nelson-Test sind in der Durchführung aufwendig und nicht zur Automatisierung geeignet. Da Antikörper gegen nichtpathogene Treponemen auch bei Gesunden vorkommen und infolge Antigenverwandtschaft mit *Treponema pallidum*-Sonikatantigen Kreuzreaktivität zeigen, erfordert die Verwendung von *Treponema pallidum*-Vollantigen eine Serumabsorption mit Präparationen aus *Treponema phagedenis.*

Die ELISA-Systeme auf der Basis von *Treponema pallidum* als Vollantigen erfüllen zwar die Qualitätskriterien in Bezug auf Sensitivität und Spezifität, sind aus Kostengründen jedoch nicht geeignet, die weitverbreitete Zweistufendiagnostik TPHA, VDRL-Test oder RPR-Test in Kombination mit dem FTA-ABS abzulösen.

In der EP 0 391 330 B1 wird bereits festgestellt, daß die auf dem Markt befindlichen Tests den Anforderungen an Screening-Tests nicht entsprechen, auch hinsichtlich des technischen Aufwandes und der Testbewertung. Dort wird vorgeschlagen, dem dadurch entgegenzuwirken, daß die Antigene an eine Festphase gebunden werden, und zwar entweder irreversibel direkt oder über einen nicht immunchemisch bindenden Spacer.

Aufgabe der Erfindung ist es demgegenüber, ein Verfahren und ein Mittel zum Nachweis von Antikörpern gegen *Treponema pallidum* vorzuschlagen, die eine Verbesserung dieser Situation erlauben.

Diese Aufgabe wird durch ein Verfahren gemäß Anspruch 1 gelöst, bei dem eine Auswahl von rekombinanten Antigenen genamplifiziert und kloniert wird, anschließend diese genamplifizierten und klonierten Antigene in Wirtsvektorsystemen exprimiert und dann gereinigt werden, danach die gereinigten Antigene einzeln oder in Kombination an eine feste Phase gebunden werden, danach die an die feste Phase gebundenen Antigene einer Reaktion mit einem auf Antikörper hin zu untersuchenden Material ausgesetzt werden, und danach die mittels Antigen-Antikörperreaktion gebundenen Antikörper aus dem zu untersuchenden Material mittels eines Detektionssystems qualitativ und/oder quantitativ festgestellt werden.

Durch ein derartiges Verfahren entsteht erstmals ein hinsichtlich des technischen Aufwandes vertretbares Verfahren, das zugleich im Ergebnis ein erforderliches Höchstmaß an Sensitivität und Spezifität bei der Bestimmung von Antikörpern gegen *Treponema pallidum* liefert.

Ein erfindungsgemäßes Mittel aus rekombinanten Antigenen zur Durchführung eines Verfahrens zum Nachweis von Antikörpern gegen *Treponema pallidum* besteht aus 17 kD-Antigen, 47 kD-Antigen und TmpA.

Eine Kombination aus diesen drei Antigenen führt zu einer hohen diagnostischen Sensivität in der serologischen Syphilisdiagnostik. Sie ermöglicht den simultanen Nachweis der Antikörper gegen Antigene, gegen die in allen Stadien der Syphilis hohe Antikörperkonzentrationen zu finden sind.

Bevorzugt ist bei bestimmten Anwendungsfällen die Hinzunahme bestimmter rekombinanter Antigene, und zwar entweder einzeln zu den drei vorgenannten oder auch in Gruppen von zwei oder mehr zu den drei vorgenannten. Es handelt sich dabei um BMP, das 34 kD - Antigen, TmpC und Tp4.

Eine Hinzunahme dieser rekombinanten Antigene ist in bestimmten Anwendungsfällen bevorzugt, da durch zusätzliche Erfassung von Antikörpern gegen *Treponema-pallidum-*spezifische Epitope dieser rekombinanten Antigene eine weitere Verbesserung der Sensitivität des serologischen Syphilisnachweises erreicht werden kann.

Zur Vermeidung von Spezifitätsverlusten ist die optionale Hinzunahme dieser rekombinanten Antigene an folgende Voraussetzungen gebunden:
1. Unspezifische Bindungen müssen minimiert werden.
2. Kreuzreagierende Antikörper im Untersuchungsmaterial müssen entfernt oder neutralisiert werden, z.B. durch Absorption.

Bei einer kombinierten Verwendung rekombinanter Antigene ist für die jeweilige Ausführungsform sicherzustellen, daß Antikörper gegen jedes einzelne Antigen einen substantiellen Anteil am detektierten Signal (Extinktionszunahme, Chemilumineszenz, Radioaktivität, Fluoreszenz etc.) hervorrufen.

Vorschläge zur Klonierung von *Treponema pallidum-*DNA anstelle der mangelnden *in vitro*-Kultivierbarkeit sind zwar schon gemacht worden, allerdings sind dabei keine kommerziell nutzbaren diagnostischen Systeme entstanden, die die Sensitivität und Spezifität der bisherigen Syphilisdiagnostik erreichen konnten. Bekannt sind daher lediglich Spekulationen aus der Literatur, jedoch keine Berichte über Verfahren zur Serodiagnostik der Syphilis unter Anwendung einer Kombination von rekombinanten Antigenen.

Es stellte sich heraus, daß ein prinzipielles Problem der Serodiagnostik mit Einzelantigenen darin besteht, daß die Höhe der Antikörperspiegel für die einzelnen Antigene sowohl vom Krankheitsstadium abhängt, als auch personenabhängige, individuelle Unterschiede auftreten. Ein kombinierter Antikörpernachweis gegen mehrere diagnostisch relevante Antigene, also eine Auswahl derselben, kann daher die Sensitivität und Spezifität der serologischen Diagnostik steigern.

In den Unteransprüchen sind weitere bevorzugte, erfindungsgemäße Mittel angegeben.

Ein weiterer entscheidender Faktor für Testsysteme mit rekombinanten Proteinen ist deren Reinheit. Bei Verwendung von rekombinanten Antigenen aus Expressionssystemen mit *E. coli* ist eine möglichst vollständige Entfernung der Wirtsproteine erforderlich, da sonst natürliche Antikörper gegen dieses Darmbakterium im Testsystem nachgewiesen werden. Die Herstellung der rekombinanten Proteine mit Klonierungsstrategie, Expression und Reinigung der verwendeten rekombinanten Proteine ist deshalb von erheblicher Bedeutung für das jeweilige Testverfahren.

Das erfindungsgemäße Verfahren basiert auf der Reaktion von spezifischen Antikörpern aus insbesondere Körperflüssigkeiten wie Serum, Plasma oder Liquor mit rekombinanten Antigenen von *Treponema pallidum*, die an eine feste Phase gebunden sind. Durch spezifische Antigen-Antikörperreaktion gebundene Antikörper werden bevorzugt durch einen sekundären markierten Antikörper nachgewiesen. Dabei sind radioaktive Markierungen, Markierungen mit Fluoreszenzfarbstoffen oder Enzymen möglich. Gemessen wird die gebundene Radioaktivität (Prinzip des Radioimmunassays), die Fluoreszenz (Fluoreszenzimmunoassay), die Enzymaktivität durch Substratfarbreaktion (colorimetrischer Immunoassay) oder Substratreaktion mit Chemilumineszenz (Chemilumineszenzimmunoassay). Exemplarisch vorgestellt werden Verfahren mit colorimetrischem und mit Chemilumineszenznachweis.

Die Antigene werden durch verschiedene Prinzipien an eine feste Phase gebunden. Mögliche Bindungsprinzipien sind die adsorptive Bindung, kovalente Bindung oder eine Bindung durch Reaktion mit einem bereits festphasegebundenen spezifischen Fangantikörper. Als Trägermaterial können Mikropartikel, Kugeln, Stäbchen, Röhrchen und Mikrotestplatten oder poröse Materialien verwendet werden. Exemplarisch vorgestellt werden Testsysteme mit Mikrotestplatten, an die die Antigene adsorptiv gebunden wurden sowie ein Nachweisverfahren mit kovalenter Bindung der Antigene an paramagnetische Partikel.

Die Verwendung dieser Verfahren erfolgt zusammen mit einer Auswahl von rekombinanten Antigenen von *Treponema pallidum.* Die Gene für das 17 kD-Antigen, für das 47 kD-Antigen, TmpA, TmpC und BMP, das 34 kD-Antigen und Tp4 wurden aus *Treponema-pallidum-*DNA durch Polymerasekettenreaktion amplifiziert, in den Plasmidvektor pQE-30 inseriert und in *E. coli* M15[pREP4] transformiert. Das verwendete Wirts-Vektorsystem erlaubte eine induzierbare Expression der rekombinanten Proteine mit nachfolgender affinitätschromatographischer Reinigung der rekombinanten Proteine, die dann an die feste Phase gebunden wurden.

Gegenstand der Erfindung ist weiterhin das Verfahren zur Auswahl der rekombinanten Antigene mit Eignung für die Serodiagnostik der Syphilis durch Prüfung des Antikörpergehaltes in Patientenseren der verschiedenen Stadien und der kombinierte Nachweis von Antikörpern gegen ausgewählte Antigene von *Treponema pallidum.*

Im folgenden werden bevorzugte Ausführungsformen der Erfindung anhand der beigefügten Figuren beschrieben. Es zeigen
- **Figur 1**: einen Westemimmunoblot mit dem Antigenprofil von *Treponema pallidum*;
- **Figur 2**: ein Coomassie-gefärbtes Gel und einen Westemimmunoblot mit rekombinanten Antigenen;
- **Figur 3**: einen colorimetrischen ELISA zur Abhängigkeit der optischen Dichte und des Positiv/Negativquotienten von der Konjugatverdünnung;
- **Figur 4**: einen colorimetrischen ELISA zur Abhängigkeit der optischen Dichte von der Serumverdünnung;
- **Figur 5**: einen Chemilumineszenz-ELISA zur Abhängigkeit der Chemilumineszenz von der Serumverdünnung;
- **Figur 6**: einen Chemilumineszenz-ELISA zur Ermittlung des Positiv/Negativquotienten in Abhängigkeit von der Serumverdünnung;
- **Figur 7**: einen cotorimetrischen ELISA und einen ChemilumineszenzEUSA zum Nachweis von Antikörpem gegen *Treponema pallidum* in Patientenseren; und
- **Figur 8**: einen Chemilumineszenz-ELISA zum Nachweis von Antikörpern gegen *Treponema pallidum* in verschiedenen Syphilisstadien.

Anhand der Figuren werden im folgenden beispielhaft Klonierung, Expression und Reinigung der *Treponema pallidum*-Antigene erläutert.

Für ausgewählte Antigene von *Treponema pallidum* wurde unter Verwendung spezieller Klonierungsprimer eine Genamplifikation durch die Polymerasekettenreaktion (PCR) an *Treponema pallidum-*DNA durchgeführt. Das resultierende PCR-Produkt wurde durch die Restriktionsendonukleasen *Bam*HI und *Sal*I geschnitten und nachfolgend durch Ligation in den *Bam*HI/*Sal*I-geschnittenen Expressions-Plasmidvektor pQE-30 inseriert. Danach wird *E.coli* M15[pREP4] transformiert. Plasmidvektor und Wirt sind beschrieben in "The QIAexpressionist", herausgegeben von der Qiagen Inc., Chatsworth, CA 91311, U.S.A, von der auch Vektor und Wirt erhältlich sind, ferner wird auf die EP 0 282 042 B1 Bezug genommen.

In den so erhaltenen Klonen läßt sich die Expression der rekombinanten Antigene gezielt induzieren. Eine affinitätschromatographische Reinigung wird durch das verwendete Expressionssystem ermöglicht und liefert die rekombinanten Antigene in ausreichender Reinheit für diagnostische Tests.

Es folgt eine Vorauswahl starker Immunogene von *Treponema pallidum.* Im Verlauf der Syphilis werden Antikörper gegen eine Vielzahl von *Treponema pallidum*-Proteinen gebildet (Norris, Microbiol. Rev. 57, 750-775 (1993)). Zur Identifizierung starker Immunogene wird ein Westemimmunoblot durchgeführt. Dazu wird Vollantigen von *Treponema pallidum* in der Sodium Dodecylsulfat Polyacrylamide Gel Electrophoresis (SDS-PAGE) aufgetrennt und die Proteine auf Nitrozellulose überführt (Westemblot).

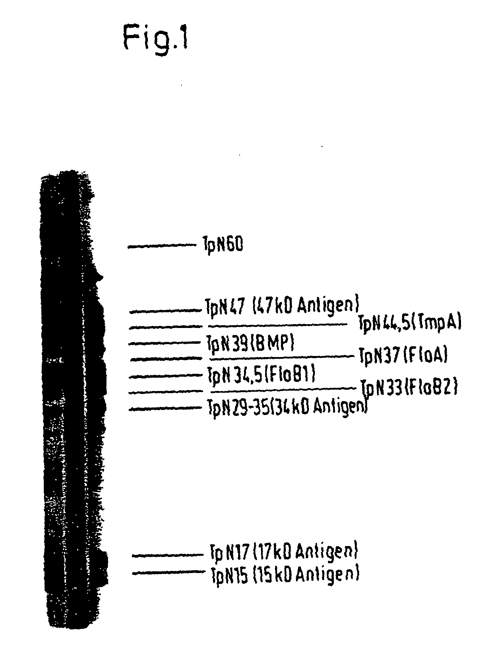

Das Ergebnis zeigt **Figur 1.** Nach Inkubation mit einem Poolserum von Patienten mit Sekundärsyphilis (Bahn 1), mit einem Primärsyphilispatientenserum (Bahn 2) und einem Sekundärsyphilispatientenserum (Bahn 3) gefolgt von Inkubation mit einem Meerrettichperoxidase-konjugierten Antihumanglobulin wurden durch das ECL™ - Detektionssystem (Amersham) immunoreaktive Antigene sichtbar gemacht. Von oben nach unten zeigen die Hinweispfeile TpN60, TpN47 (47 kD-Antigen), TpN44,5 (TmpA), TpN39 (BMP), TpN37 (FlaA), TpN34,5 (FlaB1), TpN33 (FlaB2), TpN29-35 (34 kD-Antigen), TpN17 (17 kD-Antigen) und TpN15 (15 kD-Antigen). Die vor den Klammern stehenden Bezeichnungen sind dabei die entsprechend einer neueren von Norris (s.o.) eingeführten Nomenklatur, die in den Klammem entsprechen der vorwiegend angewendeten.

Dabei zeigten Patientenseren mit aufgetrenntem *Treponema pallidum-*Vollantigen starke Reaktivität mit Banden, die dem 15 kD-Antigen, dem 17 kD-Antigen, dem 34 kD-Antigen, den Endoflagellenantigenen (FlaB1, FlaB2, FlaA), dem BMP, dem TmpA und dem 47kD-Antigen entsprechen.

Wegen bekannter Antigenverwandtschaft zu homologen Proteinen anderer Treponemenspecies werden Endoflagellenantigene nicht ausgewählt. Die übrigen Antigene werden kloniert und mit Ausnahme des 15 kD-Antigens exprimiert.

Die Klonierung der *Treponema pallidum*-Antigene verläuft wie folgt: *Treponema pallidum* wird aus infizierten Kaninchenhoden gewonnen, und die genomische DNA wird durch eine modifizierte Proteinase K/ SDS-Methode isoliert. Die Genamplifikation durch Polymerasekettenreaktion (PCR) erfolgt mit Hilfe spezifischer Primer (Tabelle 1), so daß Restriktionsendonukleaseschnittstellen (*Bam*HI am 5'-Ende, *Sal*I am 3'-Ende) den Einbau der PCR-Amplifikate in die entsprechende Klonierungsstelle des Vektors pQE-30 ermöglichen.

Tabelle 1 am Ende der Beschreibung zeigt die Klonierungsprimer für die Genamplifizierung der *Treponema pallidum*-Antigene. In der Tabelle sind in fett die Schnittstelle der Restriktionsendonuklease wiedergegeben, kursiv ist die erste vom PCR-Amplifikat kodierte Aminosäure dargestellt (TGT und TGC sind Zystein, CAG ist Alanin und AAG ist Lysin); das Stopp-Codon ist unterstrichen markiert.

Die PCR wird in einem Endvolumen von 100 µl durchgeführt und enthält 50 mM KCI, 10 mM Tris HCI pH 8.3, 1,5 mM MgCl2, Desoxyribonukleotid-Mix (je 200 µM für ATP, GTP, TTP, CTP ), je 100 pmol Klonierungsprimer 1 und 2 (Tabelle 1), 1 ng chromosomale *Treponema pallidum-*DNA bzw. 20 ng Plasmid-DNA mit inserierter *Treponema pallidum-*Gensequenz und 1 Einheit Taq-Polymerase (Pharmacia). Der Ansatz wird mit 100 µl Öl überschichtet (Mineral Oil, SIGMA). Die Reaktionen werden im Thermocycler (Landgraf) durchgeführt (Denaturierung 60 s, 94 °C, Annealing 120 s, 50 °C, Polymerisation 120 s, 72 °C). Die Größe der PCR-Produkte werden in der Agarosegelelektrophorese überprüft.

Restriktion und Ligation von Vektoren und PCR-Produkten erfolgen nach Standardprotokoll der Hersteller der Enzyme. Die In-Gel-Ligation wird nach dem Protokoll des Herstellers der niedrigschmelzenden Agarose durchgeführt (Seaplaque GTG, FMC). Beim Einsatz der Restriktionsendonukleasen, der alkalischen Phosphatase und der T4 DNA-Ligase (Pharmacia) werden die Hinweise des Herstellers berücksichtigt. Für die Reinigung von DNA-Fragmenten wird das Glass Max System (Gibco BRL) verwendet. Der korrekte Einbau der PCR-Amplifikate wird durch Molekulargewichtsbestimmung in der Agarosegelelektrophorese und durch DNA-Teilsequenzierung bestätigt.

Es folgt die Expression der *Treponema pallidum*-Antigene. Durch die angewendete Klonierungsstrategie unter Verwendung des Plasmidvektors pQE-30 werden Expressionssysteme für rekombinante *Treponema pallidum*-Antigene erhalten, die wie folgt charakterisiert sind: Auf ein Promotor-Operator-Element, das aus dem Promotor des Bakteriophagen T5, zwei *lac*-Operatorsequenzen und einer synthetischen Ribosomen-Bindungsstelle besteht (Stüber et al., in: Lefkovits / Pernis, Immunological Methods Volume IV, 121-152 (1990)), folgen Startcodon, Sequenzen für 6 aufeinanderfolgende Histidinreste, ein Polylinker zur Insertion proteincodierender Sequenzen sowie ein vektorcodierter Transkriptionsterminator. Bei Verwendung des Expressionsvektors pQE-30 sind die rekombinanten Proteine durch eine N-terminale Hexahistidinsequenz modifiziert. Der Laktoserepressor zur Kontrolle der Genexpression wird auf dem Plasmid pREP4 codiert, das im Wirtsstamm M15 in mehreren Kopien vorliegt und über seine Kanamycinresistenz selektiert wird. Als Induktor der Genexpression dient das synthetische Lactosederivat IPTG.

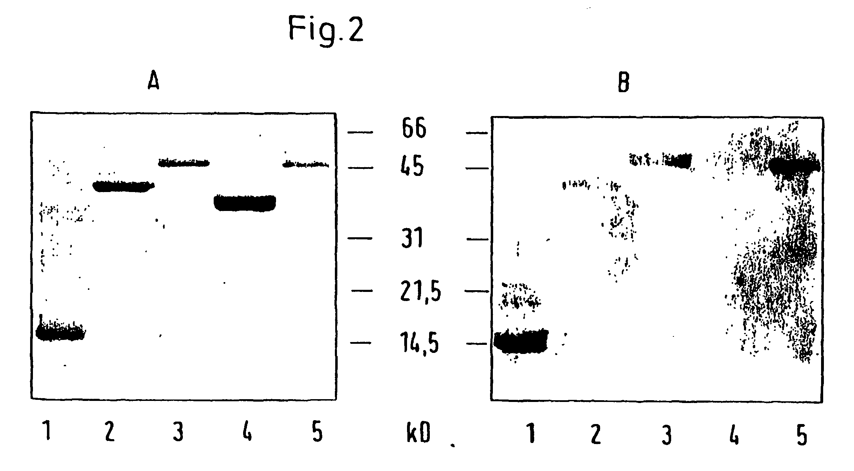

**Figur 2** zeigt Expression und Immunoreaktivität der Fusionsproteine 17 kD-Antigen, 47 kD-Antigen, BMP, TmpA, TmpC. Zur Expression der rekombinanten Antigene werden Bakterien des Stammes M15 mit den entsprechenden rekombinanten Plasmiden angezüchtet und die Genexpression durch Zugabe von 2 mM IPTG in der logarithmischen Wachstumsphase induziert. Nach dreistündiger Weiterinkubation werden die Zellen geerntet, die Fusionsproteine über Ni-NTA-Sepharose isoliert und in der SDS-PAGE aufgetrennt. Für alle Fusionsproteine kann im Coomassie - gefärbten Gel (linke Bildhälfte) jeweils eine distinkte Proteinbande sichtbar gemacht werden.

Der Westemblot (rechte Bildhälfte) zeigt bei den entsprechenden Klonen Immunoreaktivität der rekombinanten Proteine 17 kD-Antigen (Spalte 1), BMP (Spalte 2), 47 kD-Antigen (Spalte 3) und TmpA (Spalte 5). Für das TmpC (Spalte 4) kann keine Immunoreaktivität detektiert werden.

Für die konstruierten Expressionssysteme mit Ausnahme für das 15 kD-Antigen wird bereits nach einer Stunde eine starke Genexpression nachgewiesen. Für nachfolgende Reinigung werden Kulturen verwendet, die drei Stunden mit 2 mM IPTG induziert werden. Durch SDS-PAGE und Coomassie-Färbung wird das erwartete Molekulargewicht bestätigt und im Westemblot die Reaktivität der rekombinanten Antigene mit Syphilisserum nachgewiesen (Figur 2). Für das 47 kD-Antigen, das 17 kD-Antigen, BMP und TmpA ist jeweils eine Bande deutlich erkennbar. Für das 17 kD-Antigen ist eine weitere sehr schwache immunoreaktive Bande mit einem Molekulargewicht von 35 kD sichtbar, die auf eine Dimerbildung zurückzuführen sein könnte. Das TmpC zeigt keine und das 34 kD-Antigen nur eine schwache Immunoreaktivität.

Die sechs aufeinanderfolgenden Histidin-Aminosäurereste der rekombinanten Fusionsproteine gestatten eine Reinigung der Antigene nach dem Prinzip der immobilisierten Metall-Chelat-Affinitätschromatographie (Hochuli et al., J. Chromatogr. 411, 177-184 (1987)). Der verwendete Chelat-Ligand NTA (Nitrilotriacetic acid) hat vier Bindungsstellen, welche vier der sechs möglichen Bindungen des komplexbildenden Nickelions besetzen. Der mit Metallionen beladene Chelat-Ligand (Ni-NTA, Hoffmann-La Röche) ist an Sepharose CL-6B immobilisiert. Die übrigen zwei freien Bindungsstellen des Nickelions reagieren mit dem 6xHistidin-Affinitätsende am N-terminalen Ende des rekombinanten Proteins. Die Elution der rekombinanten Proteine erfolgt durch Protonierung der Histidin-Aminosäurereste im sauren pH-Bereich (etwa bei pH 5.8) und Dissoziation vom Ni-NTA-Liganden. Die Reinigung der rekombinanten *Treponema pallidum*-Antigene wird nach dem Protokoll des Herstellers (QIAGEN, s.o.) aus dem Pellet von 500 ml induzierter Bakterienkultur unter denaturierenden Bedingungen durchgeführt. Die Ausbeute liegt zwischen 5 bis 68 mg Protein / I induzierter Kultur. Die densitometrisch bestimmte Reinheit schwankt von 70 bis 99 %.

Es wird im folgenden die Bindung von *Treponema pallidum-*Antigen an eine feste Phase und der Nachweis spezifischer Antikörper anhand einiger Beispiele diskutiert.

### Beispiel 1: Adsorptive Bindung von rekombinantem Treponema pallidum-Antigen an Mikrotiterplatten und colorimetrischer Enzymimmuntest (col. EIA)

Mikrotiterplatten mittlerer Bindungskapazität (F-Form, Greiner) wurden 18 Stunden bei 4 °C mit 100 µl Antigen in 0,1 M Bicarbonatpuffer beladen (0,04 M Na₂CO₃, 0,06 M NaHCO₃, pH 9.6). Die Antigenkonzentration betrug 1 µg/ml. Die beladenen Platten wurden dreimal mit 200 µl Waschpuffer (PBS, 0,05 % Tween 20) gespült, um nicht gebundenes Antigen zu entfernen. Anschließend wurde eine einstündige Blockierung der freien Bindungsstellen auf der Plastikoberfläche der Mikrotiterplatte mit 5%igem Magermilchpulver in Waschpuffer durchgeführt, der sich ein dreifacher Waschvorgang anschloß. Danach wurde bei Raumtemperatur eine zweistündige Inkubation mit 100 µl Patientenserum in Waschpuffer in verschiedenen Verdünnungen durchgeführt. Es folgten drei Waschschritte mit jeweils 200 µl Waschpuffer. Ein Anti-human-lgG-AP-Konjugat (γ-kettenspezifisch) wurde nach Angaben des Herstellers (SIGMA) in Waschpuffer verdünnt. Jeweils 100 µl Konjugat wurden in die Vertiefungen der Mikrotiterplatte gegeben und zwei Stunden bei Raumtemperatur inkubiert. Nach dreimaligem Waschen wurden 200 µl des Enzymsubstrates *p*-Nitrophenylphosphat in 0,2 M Tris (*p*-NPP-Substrattablettenset, SIGMA) zugegeben und bei Raumtemperatur im Dunkeln inkubiert. Nach 30 Minuten wurde die Reaktion durch Zugabe von 50 µl 3 N NaOH abgestoppt. Die Extinktion wurde gegen den Substrat-Blank bei einer Wellenlänge von 405 nm im Mikrotiterplattenphotometer Anthos gemessen.

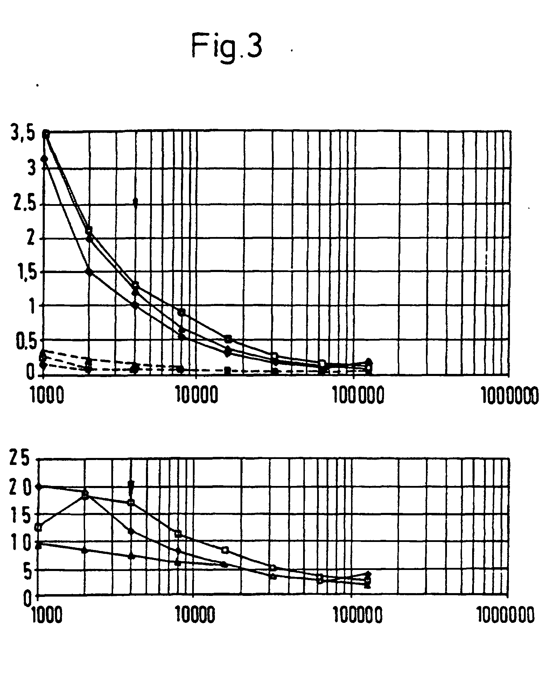

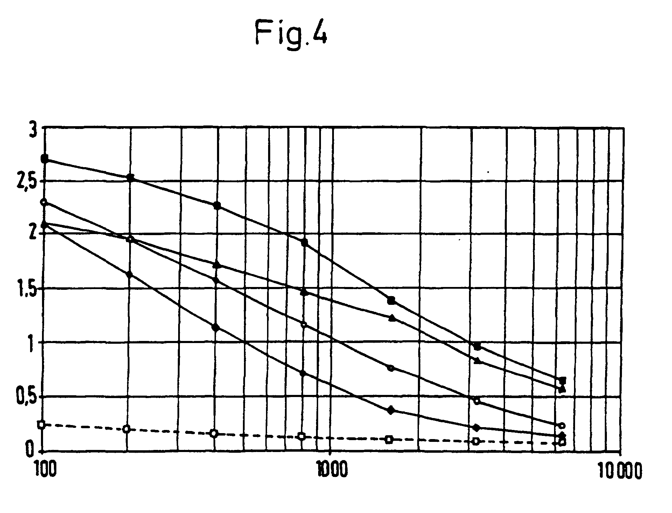

Zur Testoptimierung wurden Schachbrettitrationen durchgeführt und als Standardbedingungen eine Verdünnung des sekundären Antikörpers von 1:4.000 und des Untersuchungsserums von 1:400 ausgewählt (Figuren 3 und 4). Bei höheren Verdünnungen war eine Verringerung des Quotienten aus Extinktionen eines Sekundärsyphilispoolserums und einem Negativserum (P/N-Quotient) zu verzeichnen. Für das 17 kD-Antigen, das 47 kD-Antigen und das TmpA wurden mit 17.1, 7.4 und 11.7 die höchsten P/N-Quotienten ermittelt. Deshalb wurden diese drei Antigene für einen kombinierten ELISA ausgewählt.

**Figur 4** zeigt als Abszisse die Serumverdünnung, als Ordinate OD bei 405 nm. Gezeigt ist die Abhängigkeit der Extinktionen im colorimetrischen ELISA von der Serumverdünnung nach separater Beladung der Mikrotiterplatten (mit jeweils 1 µg/ml). Ein Negativpool verläuft als unterste Kurve und ist durch rechteckige Kästchen gekennzeichnet, darüber verlaufen die Kurven von (in dieser Reihenfolge) 47 kD - Antigen, TmpA, 17 kD - Antigen sowie nach simultaner Beladung mit diesen drei rekombinaten Antigenen (jedes 1 µg/ml).

### Beispiel 2: Adsorptive Bindung von rekombinantem Treponema pallidum-Antigen an Mikrotiterplatten und Chemilumineszenz-Enzymimmuntest (CL-EIA).

Auf der Basis des ELISA-Light Kit (Tropix Inc., USA), dessen Detektionssystem auf der Chemilumineszenz bei enzymatischer Umsetzung von Dioxetanen (EP 0 275 260 B1) beruht, wurde folgendes Protokoll entwickelt:

Mikrotiterplatten (Mikrolite®, Dynatech USA) werden unter den in Beispiel 1 genannten Bedingungen beladen (Antigenlösung 1µg/ml in Bicarbonatpuffer) und nach 18stündiger Inkubation bei 4 °C dreimal gewaschen. (PBS, 0,05 % Tween 20). Nach einstündiger Blockierung (PBS, 0,5 % I-Block [Tropix Inc.]), 0,05 % Tween 20) wird viermal mit Waschpuffer gespült (0,2 % I-Block, 0,05 % Tween 20 in PBS). Die nachfolgende Inkubation mit in Waschpuffer verdünnten Patientenseren erfolgt über einen Zeitraum von einer Stunde. Nach drei Waschschritten schließt sich eine einstündige Inkubation mit 1:2.500 in Waschpuffer verdünntem Anti-human-IgG-AP-Konjugat (γ-kettenspezifisch, SIGMA) an.

Anschließend wird dreimal in Waschpuffer und zweimal in Assaypuffer (0,1 M Diethanolamin, 1mM MgCl₂, pH 10.0) gewaschen. Danach wird für 10 Minuten mit 100 µl Verstärker/ Substrat inkubiert (Assaypuffer, 10 % Sapphire, 0,2 mM CSPD, [beides Tropix Inc.]). Die Chemilumineszenz wird nach 20 Minuten im Mikrotiterplattenluminometer Lucyl (Anthos) gemessen.

Zur Testoptimierung werden Schachbrettitrationen durchgeführt und als Standardbedingungen eine Verdünnung des sekundären Antikörpers von 1:2.500 und des Untersuchungsserums von 1:800 ausgewählt.

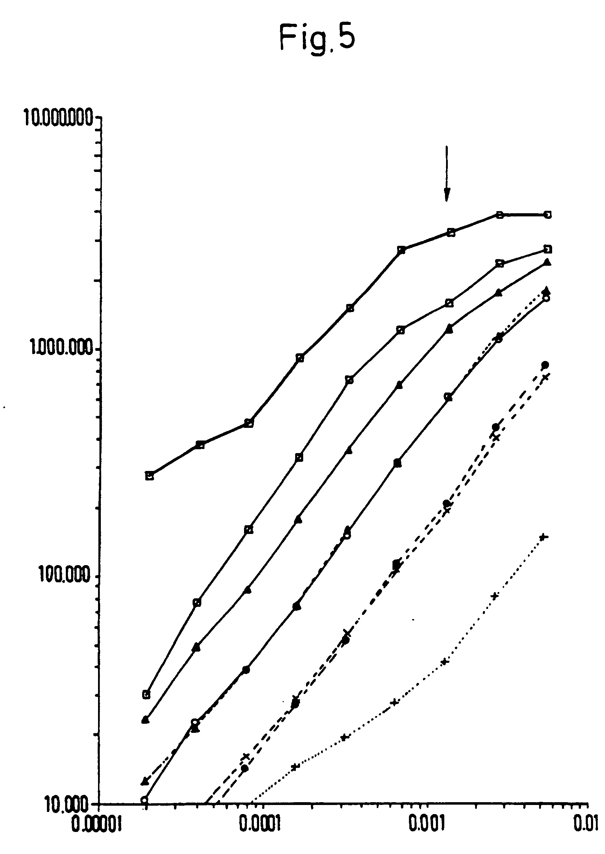

**Figur 5** zeigt die Abhängigkeit der Chemilumineszenzintensität (in Counts per Sekunde, auf der Ordinate aufgetragen) von der Antikörperkonzentration (als Serumverdünnung auf der Abszisse aufgetragen) gegen die einzelnen Antigene und die Antigenkombination TmpA, 47 kD-Antigen und 17 kD-Antigen (geprüft an Verdünnungen des Sekundärsyphilispoolserums). Von oben nach unten zeigen die Kurven die Chemilumineszenz-EIA (CL-EIA) der genannten Antigenkombination, von 17 kD-Antigen, TmpA, BMP, 47 kD-Antigen, 34 kD-Antigen, TmpC und Tp4. Sie ist über 6 Titerstufen nahezu linear. Die entsprechenden Kurven sind als Referenzkurven zur quantitativen Bestimmung von Antikörperkonzentrationen in unbekannten Untersuchungsproben sehr gut geeignet. Der große lineare Bereich zeigt die Überlegenheit des Chemilumineszenz-EIAs für quantitative Antikörperbestimmungen gegenüber dem colorimetrischen Nachweis.

Die Standardverdünnung von 1:800 ist durch einen Pfeil gekennzeichnet.

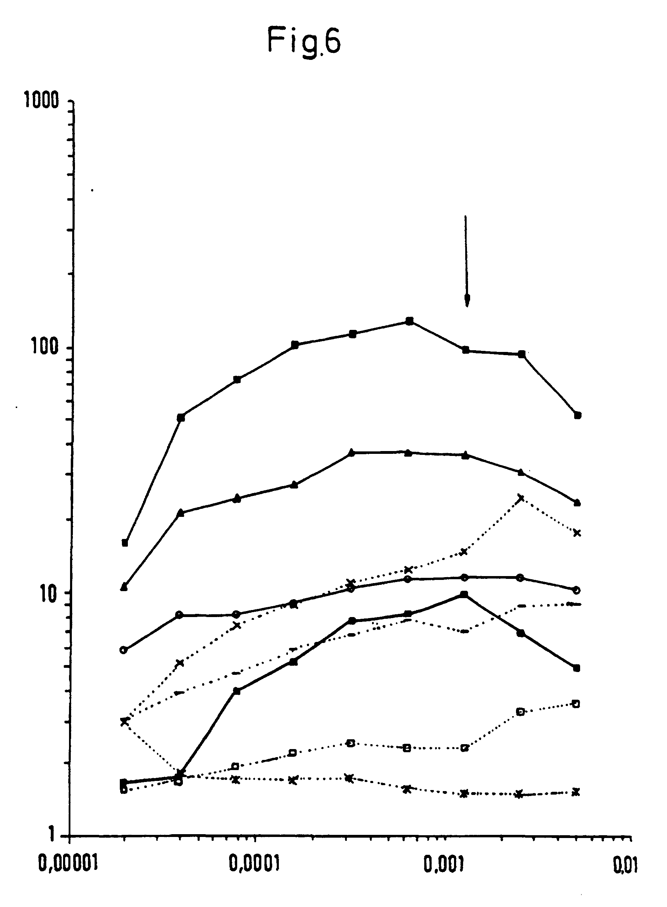

**Figur 6** zeigt den Positiv-/Negativquotienten im Chemilumineszenz - EIA aufgetragen auf der Ordinate über der Serumverdünnung auf der Abszisse. Auch hier deutet der Pfeil die Standardverdünnung von 1:800 an. Die Reihenfolge der Kurven ist hier (von oben, gesehen bei 1:800): 17 kD-Antigen - CL - EIA, TmpA, BMP, 47 kD-Antigen, Antigenkombination (wie vor), 34 kD-Antigen, TmpC, Tp4. Dieser Quotient erlaubt weitere Rückschlüsse für den diagnostischen Einsatz der rekombinanten Proteine.

Die Chemilumineszenztests mit Tp4 und TmpC sind jedoch nicht zum Einsatz in der serologischen Syphilisdiagnostik geeignet, da die Antikörperkonzentrationen gemessen an der Höhe der Chemilumineszenz wie auch die P/N-Quotienten (1,5; 2,2) zu niedrig sind. Wegen des vergleichsweise niedrigen P/N-Quotienten von 6,7 ist das 34 kD-Antigen weniger zum diagnostischen Einsatz geeignet. Für das 17 kD-Ag, TmpA, das 47 kD-Antigen und BMP wurden im entsprechenden Chemilumineszenz-EIA die höchsten Chemilumineszenzwerte gemessen. Die P/N-Quotienten lagen bei 94 für das 17 kD-Ag, 35 für das TmpA, 11 für das 47 kD-Ag und 14 für BMP (Serumverdünnung 1:800, Figur 6).

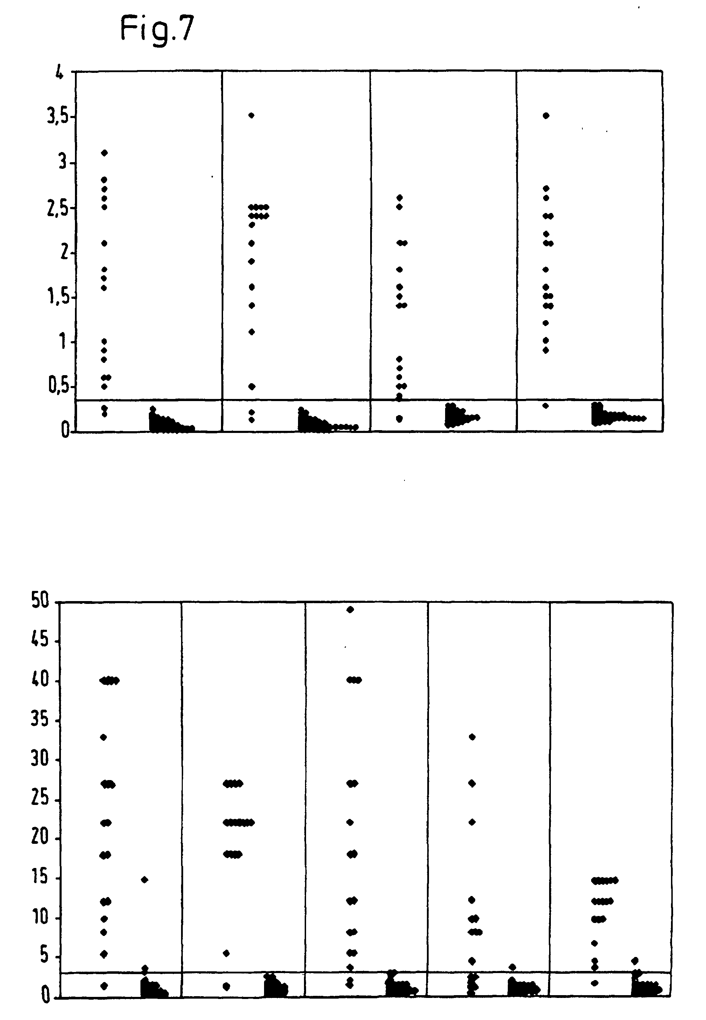

Mit dem 17 kD CL-EIA, dem TmpA CL-EIA, dem 47 kD-Ag CL-EIA und dem BMP CL-EIA und im CL-EIA mit der Kombination aus 17 kD-Antigen, 47 kD - Antigen und TmpA werden Seren von Patienten der verschiedenen Syphilisstadien untersucht. Die Chemilumineszenzwerte werden dabei auf ein Vielfaches eines mitgeführten negativen Serumpools umgerechnet. Die Ergebnisse sind in **Figur 7** dargestellt. Zur qualitativen Auswertung der Tests werden als Grenzwert die dreifache Standardabweichung vom Blutspendermittelwert (n=38) festgelegt.

Die Ergebnisse im Chemilumineszenz-EIA sind in Tabelle 2 am Ende der Beschreibung zusammen mit den Ergebnissen im colorimetrischen Test den Ergebnissen im CMT, dem TPHA-Test und dem FTA-ABS-Test gegenübergestellt. Untersucht wurden Seren von Syphilispatienten mit Primärsyphilis (LI), Sekundärsyphilis (LII), Tertiärsyphilis (LIII), Lues latens (LLa). Die Ergebnisse im colorimetrischen Test (col.EIA) wurden in Extinktionseinheiten, für den chemiluminometrischen Test (CL-EIA) in relativen Einheiten angegeben (als Vielfaches der Chemilumineszenz eines negativen Referenzserums). Seren unterhalb des definierten Grenzwertes (Mittelwert plus drei Standardabweichungen von 37 bis 42 Blutspenderseren) sind markiert. Insgesamt ist eine Befundkorrelation in der Weise vorhanden, daß für Seren mit niedrigem Titer in CMT, TPHA oder FTA-ABS auch niedrige Antikörperkonzentrationen in den Enzymimmuntests nachgewiesen werden. Dies ist besonders deutlich für Seren des latenten Syphilisstadiums. Im BMP CL-EIA liegen 8 von 18 Seren unterhalb des Grenzwertes, darunter alle untersuchten Seren im latenten Stadium der Syphilis. Für ein serologisches Syphilisscreening ist der isolierte Nachweis von Antikörpem gegen BMP nach den bisher vorliegenden Ergebnissen nicht geeignet. Die hier vorgestellten Erstbefunde lassen jedoch auf eine mögliche Korrelation der Antikörperkonzentrationen zum Krankheitsstadium und/oder der Krankheitsaktivität schließen. Sollte sich dies nach Untersuchung einer größeren Anzahl von Syphilispatienten bestätigen, könnte ein Antikörpernachweis gegen BMP z. B. als Therapiekontrolle diagnostisch verwertbar sein. Unter den 18 untersuchten Syphilisseren sind in den CL-EIAs mit dem 17 kD-Antigen und dem 47 kD-Antigen je zwei und mit TmpA drei Seren negativ. Davon ist ein Serum in allen drei Tests negativ, die anderen negativen Befunde in einem Test mit rekombinantem Antigen sind mit positiven Befunden für die anderen rekombinanten Antigene verbunden. Daraus läßt sich die Schlußfolgerung ziehen, daß eine ausreichende diagnostische Sicherheit nur durch Antikörperbestimmung gegen mehrere rekombinante Antigene zu erreichen ist.

### Beispiel 3: Kovalente Bindung von rekombinantem Treponema pallidum-Antigen und Chemilumineszenz-Enzymimmuntest

Zur kovalenten Bindung von rekombinanten *Treponema pallidum*-Antigenen an eine feste Phase wurden superparamagnetische Polystyrol-Partikel (DYNA-BEADS M-280 Tosylactivated, DYNAL Norwegen) verwendet Das Prinzip der kovalenten Bindung ist in den technischen Unterlagen ausreichend beschrieben. Weiterer wesentlicher Bestandteil dieses Verfahrens ist das Prinzip der Separation der Partikel von den jeweiligen Reaktionslösungen durch ein Magnetfeld. Im vorliegenden Beispiel wurden Eppendorfröhrchen mit den Magnetpartikeln in Reaktionslösung zur Phasentrennung in einen Magnetseparator gebracht. Dabei werden die superparamagnetischen Partikel durch das Magnetfeld eines Permanentmagneten an die Wandung gezogen und die Reaktionslösung kann durch Abpipettieren oder Absaugen entfernt werden.

Der Versuch wurde exemplarisch mit dem rekombinanten 17 kD-Antigen durchgeführt. Die Partikel wurden im Magnetseparator vom Transportmedium getrennt, zu 20 mg/ml resuspendiert, mit gleichem Volumen von rekombinantem Protein versetzt und 24 Stunden bei 37°C unter Schütteln inkubiert. Die Reaktionsbedingungen bei der dabei erfolgenden Bindung des rekombinanten Proteins an die Partikel waren 10 mg/ml Partikel, 20 µg/ml rekombinantes Protein in 0,05 M Boratpuffer pH 9,5. Anschließend wurde mit Waschpuffer (PBS, 0,2 % I-Block, 0,05 % Tween 20) dreimal 10 min, einmal 30 min und einmal über Nacht gewaschen, wobei simultan freie Proteinbindungsstellen blockiert wurden. Die beladenen Partikel wurden bei 4 °C aufbewahrt und am Tag des Tests zweimal mit Waschpuffer gewaschen und für die Versuchsansätze portioniert. Für einen Test wurden 0,05 mg Partikel mit 100 µl Patientenserum in einer Verdünnung von 1:800 in Waschpuffer über eine Stunde bei 37 °C inkubiert. Nach zweimaligem Waschen wurde mit Sekundärantikörper (1:2500 Anti-Human-Alkalische-Phosphatase-Konjugat, γ-kettenspezifisch, SIGMA) über 1 Stunde inkubiert. Anschließend wurde zweimal in Waschpuffer und zweimal in Diethanolaminpuffer (0,1 M Diethanolamin, 1mM MgCl₂, 0,02 NaN₃, pH 10.0) gewaschen. Nach Resuspendieren der Partikel in 50 µl Diethanolaminpuffer wurden diese in Mikrotiterplatten überführt, die je Kavität 50 µl Verstärker/Substratlösung enthielten. (Endkonzentrationen in 100 µl: 10 % Sapphire, 0,2 mM CSPD, [beides Tropix Inc.]). Die unter Vermittlung der alkalischen Phosphatase entstehende Chemilumineszenz bei der Umsetzung des Substrates wurde im Mikrotiterplattenluminometer Lucy1® (Anthos) gemessen. Für das rekombinante 17 kD-Antigen wurden in diesem System ein Poolserum von 73 Blutspendern mit einem Poolserum von Patienten mit Sekundärsyphilis verglichen. Bei einer Integrationszeit von 1 s je Chemilumineszenzmessung wurden als relative Counts für das Syphilispoolserum 1.481.958,5 (10 min nach Reaktionsbeginn), 1.641.317,5 (nach 20 min) bzw. 1.746.169 (nach 30 min) sowie für das Blutspenderpoolserum 7.519,5 (nach 10 min), 9.929,5 (nach 20 min) bzw. 11.629 (nach 30 min) ermittelt. Dies entspricht einem Positiv/ Negativ-Quotienten nach einer Substratinkubation von 10 min von 197, nach 20 min von 165 und nach 30 min von 150. Aus diesen Werten ist ersichtlich, daß offenbar eine geringere unspezifische Bindung von Antikörpern an die feste Phase erfolgt. Dadurch ist die analytische Sensitivität den unter Beispiel 1 und Beispiel 2 vorgestellten Systemen überlegen.

Der simultane Einsatz rekombinanter Antigene in der serologischen Diagnostik verbessert also die Möglichkeiten. Bei der Serodiagnostik von Infektionskrankheiten ist bei Nachweis verschiedener Antikörperspezifitäten gegen den Erreger prinzipiell eine Verbesserung der Sensitivität gegenüber dem Nachweis von Antikörpern gegen ein einzelnes Antigen möglich. Aus diesem Grund wird z. B. als Bestätigungstest bei der serologischen HIV-Diagnostik ein Westernimmunoblot angewendet. Dabei reagieren Antikörper aus Untersuchungsmaterial mit Erregermaterial, dessen Antigene durch eine Elektrophorese aufgetrennt und auf eine feste Phase überführt wurden. Als sicher positiv gilt der Test, wenn mehrere erregerspezifische Banden reaktiv sind. Zwingend wird der Nachweis von verschiedenen Antikörperspezifitäten zur Diagnosestellung dann, wenn der Antikörpernachweis gegen Einzelantigene nicht bei allen Kranken gelingt.

Mit der vorliegenden Erfindung wird ein Mischantigen-ELISA vorgestellt, der bei zu geringer Reaktivität des Serums gegen ein oder mehrere Antigene durch Nachweis von Antikörpern gegen die anderen Antigene einen positiven Testausfall ergibt. Bei der Untersuchung von Syphilispatientenseren mit den rekombinanten Antigenen im colorimetrischen System (Beispiel 1), wie auch mit dem Chemilumineszenztest (Beispiel 2) zeigte es sich, daß einzelne Seren im Einzelantigen-ELISA negativ reagieren (Tabelle 2). Ein Serum wies negative Testergebnisse in den Enzymimmuntests mit TmpA, BMP, 47 kD-Antigen und 17 kD-Antigen sowohl im colorimetrischen Nachweissystem als auch im Chemilumineszenznachweis auf. Eine Erklärung gibt der Westemblot mit *Treponema pallidum-*Vollantigen, der nur für Tp4 deutliche Reaktivität zeigte. Stark positiv war für dieses Serum auch ein Chemilumineszenz-ELISA mit rekombinantem Tp4. Da immunologische Kreuzreaktivität des Tp4 zu Hsp60-Proteinen anderer Bakterienspezies bekannt ist, besitzt ein isolierter Antikörpemachweis gegen Tp4 geringen diagnostischen Wert und könnte auch auf eine andere Infektion zurückzuführen sein. Die übrigen Seren mit negativem Testergebnis in einem Einzelantigen-ELISA wiesen jedoch positive Tests mit den anderen getesteten rekombinanten Antigenen auf. Der Mischantigen-ELISA war in diesen Fällen positiv. Dies beweist die Überlegenheit eines Mischantigen-ELISAs mit rekombinanten Antigenen gegenüber Einzelantigen-ELISAs für einen qualitativen Nachweis der Syphilisinfektion. Die kombinierte Anwendung rekombinanter Antigene zum Nachweis einer Syphilisinfektion stellt damit einen wesentlichen Bestandteil der vorliegenden Erfindung dar.

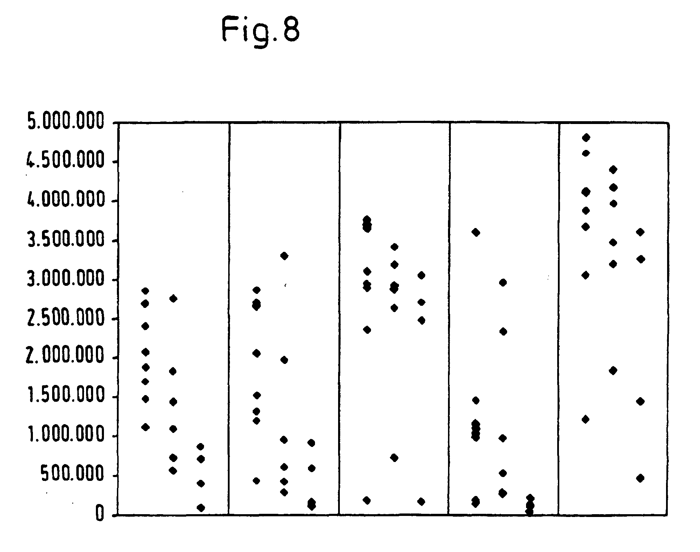

Im Einzelantigen-ELISA lagen die gemessenen Counts für hochpositive Seren auf etwa demselben Niveau (**Figur 8**), so daß die simultane Beladung der Mikrotiterplatten mit den Antigenen mit gleichen Konzentrationen erfolgte. Bei einem Test zum simultanen Nachweis von Antikörpern gegen mehrere rekombinante Antigene kommt es darauf an, daß jedes einen meßbaren Anteil am Gesamtsignal hervorruft. Die Effizienz der Bindung der rekombinanten Antigene an die Festphase kann jedoch unterschiedlich sein. Bei der Antigen-Antikörper-Reaktion mit mehreren Antigenen, die am gleichen Träger gebunden sind, könnten kompetitive Effekte ebenfalls eine Rolle spielen. Deshalb wurde ein Versuch durchgeführt, bei dem rekombinantes Antigen dem Patientenserum zugesetzt wurde. Dadurch reagieren die entsprechenden Antikörper mit dem rekombinanten Antigen in Lösung und nicht mit dem festphasegebundenen. Die Effizienz dieser kompetitiven Hemmung wurde für jede Antikörperspezifität im Einzelantigen-ELISA ermittelt und lag zwischen 91 und 99 %. Auf Grund dieses hohen Prozentsatzes konnte im Mischantigen-ELISA der gemessene Hemmeffekt näherungsweise zur Abschätzung des CL-Anteils für die AK-Bindung am einzelnen Antigen dienen. Für ein Poolserum von Patienten mit Sekundärsyphilis wurde so im Mischantigen-ELISA (Beladungskonzentration je 1 µg/ml) der Chemilumineszenzanteil durch Antikörper gegen das 17 kD-Antigen zu 52 %, TmpA-Antikörper zu 43 % und Antikörper gegen das 47 kD-Antigen zu 5 % näherungsweise ermittelt. Der Antikörpemachweis gegen das 47 kD-Antigen erscheint unterrepräsentiert. Da es sich bei dem 47 kD-Antigen um ein spezifisches Antigen von *Treponema pallidum* mit starker Immunogenität handelt, sollte der gewünschte Anteil am Gesamtsignal durch Veränderung der Konzentrationsverhältnisse der rekombinanten Antigene beim Beladen verändert und wie beschrieben geprüft werden.

Eine elegante Methode für den simultanen Nachweis der Antikörperspezifitäten stellt die Verwendung von Mikropartikeln dar. Jedes einzelne rekombinante Antigen wird in separatem Ansatz an die Mikropartikel gebunden und im Test mit Einzelantiseren die Chemilumineszenzwerte ermittelt. Mit den erhaltenen Werten läßt sich ein optimales Mischungsverhältnis der Mikropartikelpräparationen ermitteln, so daß für die größtmögliche Zahl der Syphilispatienten ein Meßergebnis über dem Grenzwert erzielt wird. Wenn Mikropartikel separat mit Antigen beladen und erst nachträglich gemischt werden, sind kompetitive Hemmeffekte sowohl bei der Antigenbindung an die Festphase als auch bei der Antigen-Antikörperreaktion ausgeschlossen. Die Funktionsfähigkeit und Überlegenheit dieses Verfahrensweges ist durch die im Beispiel 2, Beispiel 3 und dem Mischantigen-EUSA (Mikrotiterplattenverfahren) hinreichend nachgewiesen. Das beschriebene Verfahren ist geeignet zur Bindung von Antigenen, Antigenbestandteilen oder auch von Peptiden mit ausgewählten Antigendeterminanten an Mikropartikel, Bestimmung von deren diagnostischer Relevanz und deren kombinierter Einsatz für einen qualitativen Test in der Serodiagnostik der Syphilis. Auch in der Diagnostik anderer Infektionskrankheiten ist dieses Verfahren durch entsprechende Evaluierung der diagnostisch relevanten Antigene des betreffenden Erregers möglich.

Das Verfahren, bei dem verschiedene rekombinante Antigene separat an Mikropartikel als feste Phase gebunden in Testsystemen nach diagnostischen Kriterien evaluiert werden, und bei dem dann diese Mikropartikel in einem optimalen Mischungsverhältnis in einem qualitativen serologischen Test eingesetzt werden, ist von besonderem Vorteil.

Die Erfindung betrifft ein immunchemisches Verfahren zum Nachweis und zur Bestimmung von Antikörpern gegen *Treponema pallidum* in Blutserum und anderen Körperflüssigkeiten. Das beschriebene Nachweisverfahren beruht auf dem quantitativen Nachweis von Antikörpern gegen eine Auswahl rekombinanter Antigene von *Treponema pallidum* . Das vorgestellte Verfahren erlaubt einen Vergleich der Prävalenz und der Titer von Antikörpern gegen jedes *Treponema-pallidum*-Antigen bei Syphilispatienten gegenüber nichtinfizierten Patienten. Daraus lassen sich Schlüsse zur diagnostischen Relevanz des Antikörpemachweises gegen jedes untersuchte Antigen ziehen. Antikörpertiter für jedes einzelne Antigen hängen vom Krankheitsstadium ab und weisen individuelle Unterschiede auf. Es wird beschrieben, wie durch kombinierten Einsatz von mehreren rekombinanten Antigenen in einem Testverfahren eine verbesserte diagnostische Sensitivität und Spezifität erreicht werden kann. Das Verfahren des gezielten kombinierten Antikörpernachweises gegen ausgewählte Antigene zur Serodiagnostik der Syphilis ist Bestandteil dieser Erfindung. Entsprechende Testsysteme werden beschrieben. Die Anwendung der beschriebenen Verfahren führt zu einer neuen Generation von Testsystemen zum serologischen Nachweis von Syphilisinfektionen auf der Basis rekombinanter Antigene von *Treponema pallidum.*

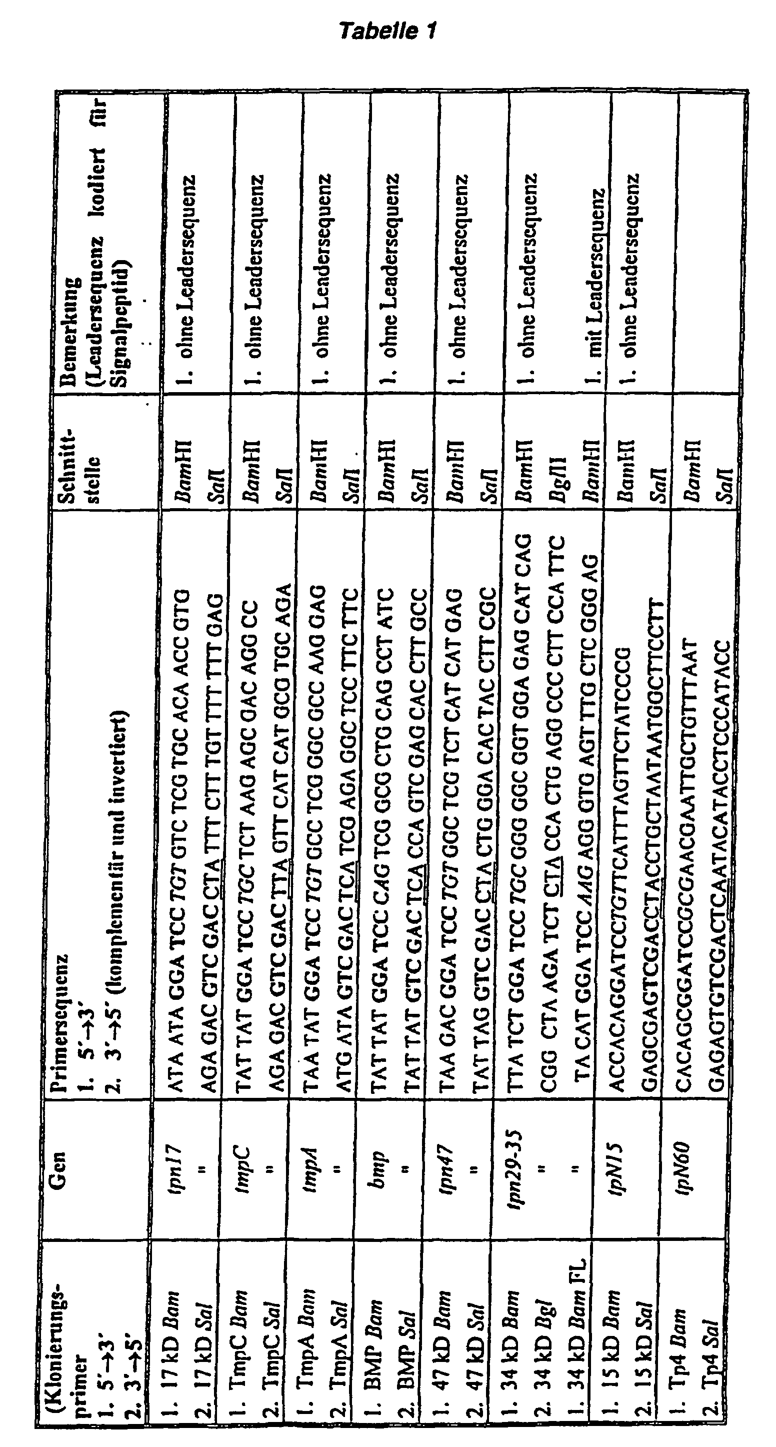

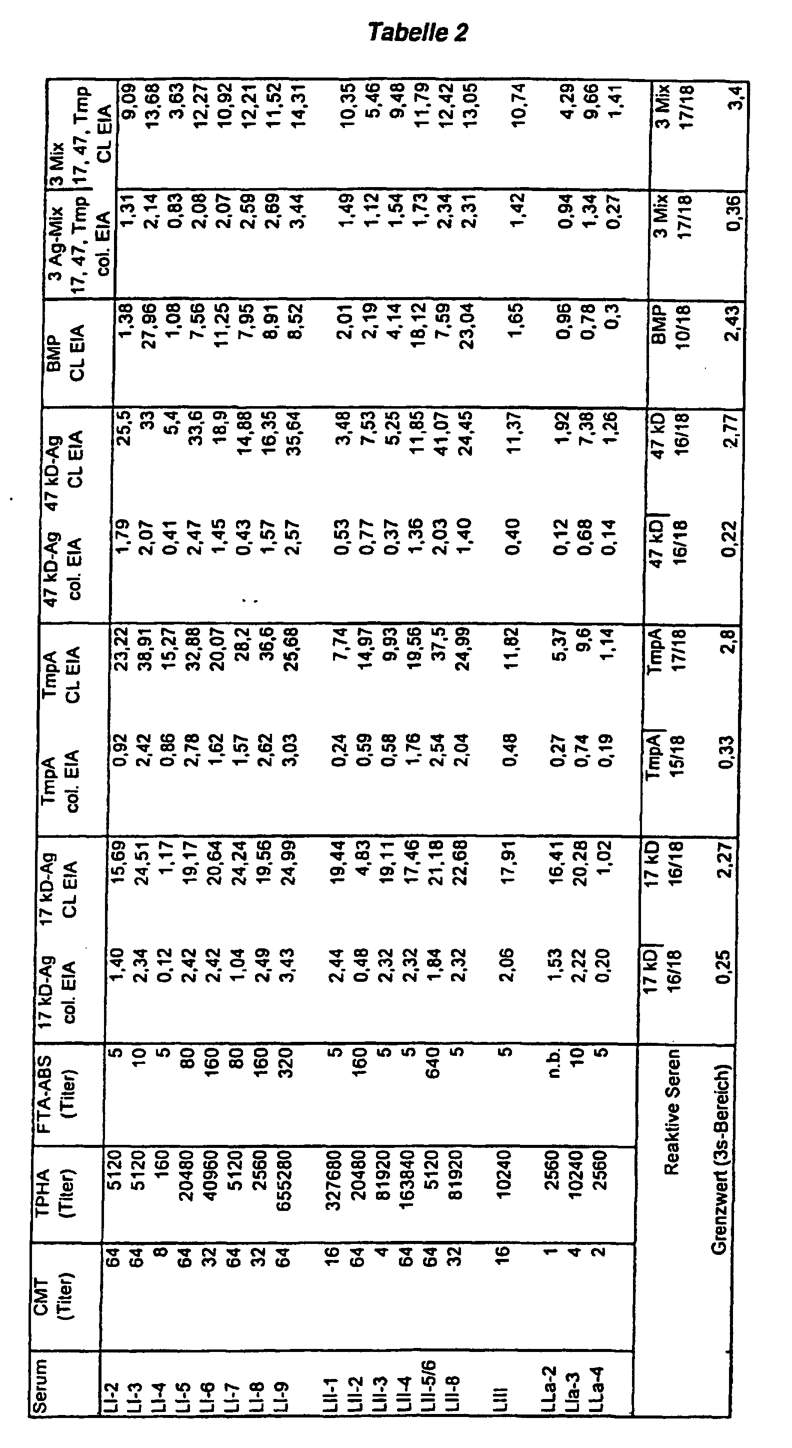

## Patentansprüche

1. Verfahren zum Nachweis von Antikörpern gegen *Treponema pallidum*, bei dem eine Auswahl von rekombinanten Antigenen genamplifiziert und kloniert wird,
anschließend diese genamplifizierten und klonierten Antigene in Wirtsvektorsystemen exprimiert und dann gereinigt werden,
danach die gereinigten Antigene in Kombination an eine gemeinsame feste Phase oder jeweib einzeln an mehrere separate feste Phasen gebunden werden,
danach die an die feste Phase oder die festen Phasen gebundenen Antigene einer Reaktion mit einem auf Antikörper hin zu untersuchenden Material ausgesetzt werden,
danach die mittels Antigen-Antikörperreaktion gebundenen Antikörper aus dem zu untersuchenden Material mittels eines Detektionssystems qualitativ und/oder quantitativ festgestellt werden, und
bei dem die Auswahl der rekombinanten Antigene zum Nachweisen von Antikörpern gegen *Treponema pallidum* besteht aus 17 kD-Antigen, 47 kD-Antigen und TmpA.

2. Verfahren nach Anspruch 1,
**dadurch gekennzeichnet,**
**daß** die Auswahl zusätzlich enthält TmpC und/oder BMP und/oder das 34 kD-Antigen und/oder Tp4.

3. Verfahren nach einem der vorstehenden Ansprüche,
**dadurch gekennzeichnet,**
**daß** die codierende DNA für die Auswahl der rekombinanten Antigene durch Polymerasekettenreaktion amplifiziert wird.

4. Verfahren nach einem der vorstehenden Ansprüche,
**dadurch gekennzeichnet,**
**daß** die amplifizierte DNA in den Plasmidvektor pQE-30 inseriert und in *E. coli* M15[pREP4] exprimiert wird.

5. Verfahren nach einem der vorstehenden Ansprüche,
**dadurch gekennzeichnet,**
**daß** die rekombinanten Antigene affinitätschromatographisch gereinigt werden.

6. Verfahren nach einem der vorstehenden Ansprüche,
**dadurch gekennzeichnet,**
**daß** die Bindung der rekombinanten, gereinigten Antigene an die feste Phase durch adsorptive oder kovalente Bindung oder über einen bereits festphasegebundenen Liganden erfolgt.

7. Verfahren nach einem der vorstehenden Ansprüche,
**dadurch gekennzeichnet,**
**daß** das zu untersuchende Material Körperflüssigkeit wie Blutserum oder Liquor oder Ascites oder Amnionflüssigkeit ist.

8. Verfahren nach einem der vorstehenden Ansprüche,
**dadurch gekennzeichnet,**
**daß** zum Nachweis der reagierenden Antikörper sekundäre markierte Antikörper eingesetzt werden.

9. Verfahren nach Anspruch 8,
**dadurch gekennzeichnet,**
**daß** die sekundären markierten Antikörper durch Fluoreszenzfarbstoff, Radioaktivität und/oder Enzyme markiert werden.

10. Verfahren nach Anspruch 9,
**dadurch gekennzeichnet,**
**daß** die Markierung des Sekundärantikörpers direkt oder indirekt über gebundene Liganden erfolgt.

11. Verfahren nach einem der vorstehenden Ansprüche,
**dadurch gekennzeichnet,**
**daß** der Nachweis in dem Detektionssystem colorimetrisch und/oder durch Messung der auftretenden Chemilumineszenz und/oder durch Fluoreszenzund/oder Radioaktivitätsmessung erfolgt.

12. Verfahren nach einem der vorstehenden Ansprüche,
**dadurch gekennzeichnet,**
**daß** bei der Bindung der rekombinanten, gereinigten Antigene an die feste Phase mehrere rekombinante Antigene gleichzeitig an die feste Phase gebunden werden.

13. Verfahren nach einem der Ansprüche 1 bis 11,
**dadurch gekennzeichnet,**
**daß** bei der Bindung der gereinigten rekombinanten Antigene an die feste Phase die Bindung jedes rekombinanten Antigens separat an einer festen Phase erfolgt und die unterschiedlich beladenen festen Phasen dann im Test gemeinsam eingesetzt werden.

14. Mittel aus rekombinanten Antigenen zur Durchführung eines Verfahrens zum Nachweis von Antikörpern gegen *Treponema pallidum*, bestehend aus 17 kD-Antigen, 47 kD-Antigen und TmpA.

15. Mittel nach Anspruch 14, zusätzlich aufweisend TmpC und/oder BMP und/oder das 34 kD-Antigen und/oder Tp4.

## Claims

1. Method for the detection of antibodies to *Treponema pallidum* in which a selection of recombinant antigens is gene-amplified and cloned,
these gene-amplified and cloned antigens are subsequently expressed in host vector systems and then purified,
the purified antigens are then bound to a common solid phase in combination or respectively to a plurality of separate solid phases,
the antigens bound to the solid phase or the solid phases are then subjected to a reaction with a material to be investigated for antibodies,
and
the antibodies bound from the material to be investigated by means of an antigen/antibody reaction are determined qualitatively and/or quantitatively by means of a detection system;
and in which the selection of the recombinant antigens for the detection of antibodies against *Treponema pallidum* consists of 17 kD antigen, 47 kD antigen and TmpA.

2. Method according to claim 1,
**characterised in that**
the selection additionally contains TmpC and/or BMP and/or the 34 kD antigen and/or Tp4.

3. Method according to one of the preceding claims,
**characterised in that**
the coding DNA for the selection of the recombinant antigens is amplified by polymerase chain reaction.

4. Method according to'one of the preceding claims,
**characterised in that**
the amplified DNA is inserted in plasmid vector pQE-30 and expressed in *E. coli* M15[pREP4].

5. Method according to one of the preceding claims,
**characterised in that**
the recombinant antigens are purified by affinity chromatography.

6. Method according to one of the preceding claims,
**characterised in that**
the recombinant, purified antigens are bound to the solid phase by adsorptive or covalent binding or via a ligand already bound to the solid phase.

7. Method according to one of the preceding claims,
**characterised in that**
the material to be investigated is a body fluid such as blood serum or liquor or ascites or amniotic fluid.

8. Method according to one of the preceding claims,
**characterised in that**
secondary, labelled antibodies are used to detect the reacting antibodies.

9. Method according to claim 8,
**characterised in that**
the secondary, labelled antibodies are labelled by fluorescent dye, radioactivity and/or enzymes.

10. Method according to claim 9,
**characterised in that**
the secondary antibody is labelled directly or indirectly via bound ligands.

11. Method according to one of the preceding claims,
**characterised in that**
detection in the detection system proceeds colorimetrically and/or by measuring the occurring chemiluminescence and/or by measurement of fluorescence and/or radioactivity.

12. Method according to one of the preceding claims,
**characterised in that**
when the recombinant, purified antigens are bound to the solid phase, two or more recombinant antigens are simultaneously bound to the solid phase.

13. Method according to one of claims 1 to 11,
**characterised in that**
when the purified recombinant antigens are bound to the solid phase, each recombinant antigen is bound separately to a solid phase and the differently loaded solid phases are then used together in the test.

14. Agent prepared from recombinant antigens for the performance of a method for the detection of antibodies to *Treponema pallidum*, which agent consists of 17 kD antigen, 47 kD antigen and TmpA.

15. Agent according to claim 14, additionally containing TmpC and/or BMP and/or the 34 kD antigen and/or Tp4.

## Revendications

1. Procédé de détection d'anticorps contre *Treponema pallidum,* dans lequel on amplifie et clone une sélection d'antigènes recombinés,
ces antigènes amplifiés et clonés sont exprimés dans des systèmes de vecteur hôte, puis purifiés,
les antigènes purifiés sont liés à une phase solide usuelle ou chaque fois individuellement, sur plusieurs phases solides séparées,
les antigènes liés à la ou aux phases solides sont mis à réagir avec un matériau à examiner en ce qui concerne les anticorps,
les anticorps liés par une réaction antigène-anticorps, du matériau à examiner, sont déterminés qualitativement et/ou quantitativement à l'aide d'un système de détection, et
dans lequel la sélection des antigènes recombinés pour la détection d'anticorps contre *Treponema pallidum* consistent en l'antigène de 17 kD, l'antigène de 47 kD et le TmpA.

2. Procédé selon la revendication 1, **caractérisé en ce que** la sélection contient en outre, le TmpC et/ou le BMP et/ou l'antigène de 34 kD et/ou le Tp4.

3. Procédé selon l'une quelconque des revendications précédentes, **caractérisé en ce que** l'ADN codant pour la sélection des antigènes recombinés est amplifié par une amplification en chaîne par polymérase.

4. Procédé selon l'une quelconque des revendications précédentes, **caractérisé en ce que** l'ADN amplifié est inséré dans le plasmide pQE-30 et exprimé dans E. coli M15[pREP4].

5. Procédé selon l'une quelconque des revendications précédentes, **caractérisé en ce que** les antigènes recombinés sont purifiés par chromatographie d'affinité.

6. Procédé selon l'une quelconque des revendications précédentes, **caractérisé en ce que** la liaison des antigènes recombinés, purifiés sur la phase solide est réalisée par liaison de type adsorption ou liaison covalente ou par l'intermédiaire d'un ligand déjà lié à la phase solide.

7. Procédé selon l'une quelconque des revendications précédentes, **caractérisé en ce que** le matériau à examiner est un liquide corporel tel que du sérum sanguin ou une liqueur ou des ascites ou le liquide amniotique.

8. Procédé selon l'une quelconque des revendications précédentes, **caractérisé en ce que** l'on met en oeuvre des anticorps secondaires marqués pour la détection des anticorps qui ont réagi.

9. Procédé selon la revendication 8, **caractérisé en ce que** les anticorps secondaires marqués sont marqués par un colorant fluorescent, par radioactivité et/ou par une enzyme.

10. Procédé selon la revendication 9, **caractérisé en ce que** le marquage des anticorps secondaires est réalisé de manière directe ou indirecte par des ligands liés.

11. Procédé selon l'une quelconque des revendications précédentes, **caractérisé en ce que** la détection dans le système de détection est réalisée de manière colorimétrique et/ou par mesure de la chimioluminescence produite et/ou par mesure de la fluorescence et/ou de la radioactivité.

12. Procédé selon l'une quelconque des revendications précédentes, **caractérisé en ce que** lors de la liaison de l'antigène recombiné, purifié sur la phase solide, plusieurs antigènes recombinés sont liés simultanément sur la phase solide.

13. Procédé selon l'une quelconque des revendications 1 à 11, **caractérisé en ce que** lors de la liaison de l'antigène recombiné, purifié sur la phase solide, la liaison de chaque antigène recombiné est réalisée séparément sur une phase solide et les différentes phases solides chargées sont alors mises en oeuvre ensemble dans le test.

14. Agent constitué d'antigènes recombinés, pour réaliser un procédé de détection d'anticorps contre *Treponema pallidum,* consistant en l'antigène de 17 kD, l'antigène de 47 kD et le TmpA.

15. Agent selon la revendication 14, présentant en outre, le TmpC et/ou le BMP et/ou l'antigène de 34 kD et/ou le Tp4.
